Europäisches Patentamt

European Patent Office

Office européen des brevets

(11) Publication number: **0 521 430 A1**

## EUROPEAN PATENT APPLICATION

(21) Application number: **92110982.3**

(22) Date of filing: **29.06.92**

(51) Int. Cl.5: **A61M 25/10**

(30) Priority: **01.07.91 IT TO910504**

(43) Date of publication of application:
**07.01.93 Bulletin 93/01**

(84) Designated Contracting States:
**AT BE CH DE DK ES FR GB GR IT LI LU MC NL PT SE**

(71) Applicant: **SORIN BIOMEDICA S.p.A.**
**Strada per Crescentino**
**I-13040 Saluggia (Vercelli)(IT)**

(72) Inventor: **Rinaldi, Stefano**
**Via Scarlatti, 4**
**I-43100 Parma(IT)**
Inventor: **Miracoli, Luigi**
**Via Fratelli Coda,, 12**
**I-16166 Genova(IT)**

(74) Representative: **Bosotti, Luciano et al**
**c/o Jacobacci-Casetta & Perani S.p.A. Via Alfieri, 17**
**I-10121 Torino(IT)**

(54) A tube structure, for example, for forming angioplasty catheters.

(57) The duct (10) in the catheter, which is intended to transport the hydraulic liquid for operating the balloon (1) of the catheter, has a crescent-shaped cross-section with end portions (12) which have small radii of curvature. These small radii of curvature favour the formation of menisci of considerable height which oppose the formation of gas-liquid fragments within the tube.

FIG. 5

EP 0 521 430 A1

Field of the invention

The present invention relates in general to tubes and has been developed with particular attention to its possible use in the field of tubes for transporting liquids through holes or ducts which have small diameters and in which capillarity phenomena thus occur.

More specifically, the invention addresses the problem that gas-liquid fragments may form in such tubes and that, in this situation, it is difficult to transport the liquid along the tube, particularly because of hydraulic and liquid-suction effects.

The invention has been developed with particular attention to its possible use in the formation of angioplasty catheters (particularly so-called PTCA or PTA catheters) to which specific reference will be made in the detailed description of a possible embodiment. The invention may, however, be used in a more general context, in any situation in which it is necessary to maintain an ability to transport a liquid along a tube by preventing the formation of gas-liquid fragments. For example, this may be the case with fuel-pipes, in which the formation of gas-liquid fragments corresponds to the phenomenon generally known as "vapour-lock".

Description of the prior art

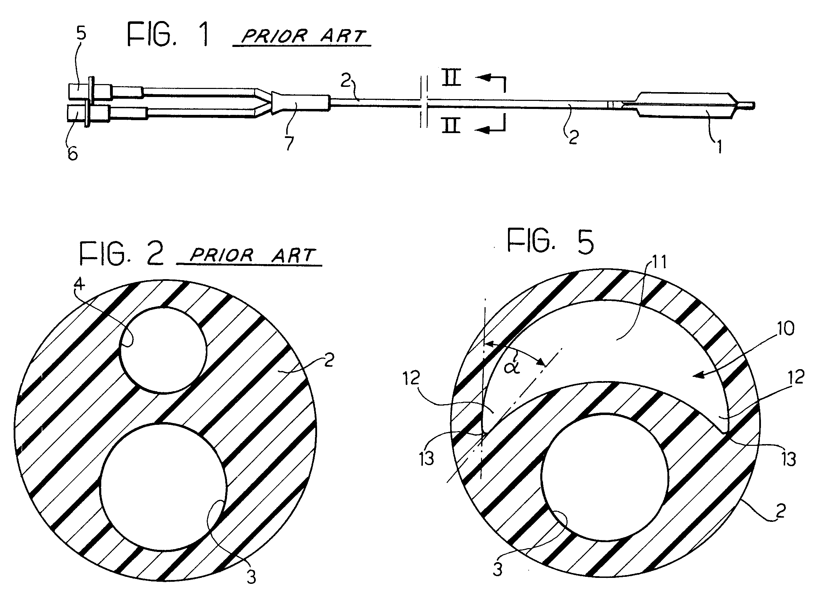

Figure 1 shows the general structure of a so-called angioplasty catheter, also generally known as a PTA (percutaneous transluminal angioplasty) catheter or a PTCA (percutaneous transluminal coronary angioplasty) catheter.

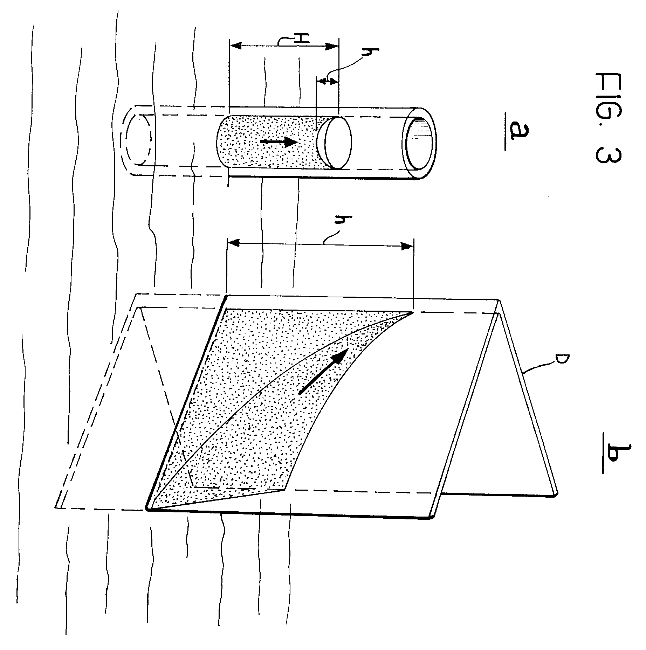

Briefly, the catheter in question is constituted essentially by an elongate head portion 1 (the actual balloon) which can be moved within the vessel which is undergoing angioplasty treatment by means of an insertion, guiding the pushing operation effected by means of a flexible tube 2 (or "shaft"), the structure of which is shown schematically in Figure 2, and which is essentially comparable to a cross-section taken on the line II-II of Figure 1.

Two ducts 3, 4 with circular cross-sections generally extend longitudinally through the flexible tube 2, which is made of any plastics material suitable for use within the body (for example, polyethylene). A metal wire (not shown) which is flexible but has a certain stiffness (for example, a platinum wire), known as the "guide wire", can extend and slide along the duct 3 (which has a diameter of the order, for example, of between 250 and 500 microns). The "guide wire" is intended to be inserted into an artery and pushed along within the artery until it has reached and passed beyond the stenotic portion of the artery which is to be expanded. The catheter is then slid along the "guide wire" (with the balloon deflated) until the balloon is in a position corresponding to the lesion; at this point the balloon is inflated in order to expand the stenotic portion of the artery permanently; finally, it is deflated and removed. The duct 4 (which has a diameter of the order of 300 microns and, in general, is not necessarily circular) is intended to act as a duct for transporting a liquid, for example, a radiographic contrast liquid (such as, the contrast media sold under the trade names Renographin or Iopamiro) so that the balloon head 1, the cavity of which communicates with the duct 4, can be inflated and deflated hydraulically (these operations usually being repeated several times). This is achieved by means of a pumping action generally effected by means of a syringe (not shown) which is fitted to a distal terminal or threaded ring, indicated 6.

The distal end of the tube 2 generally has a Y-shaped connector 7 for ensuring that the metal wire and the line for the pumping of the liquid which extend through the duct 3 and the duct 4 respectively, and are intended to be kept separate in the portion located outside the patient's body (since they have to be handled at different times and in different situations), converge precisely within the tube 2.

Naturally, angioplasty catheters with different structures are known from the prior art, for example, there are angioplasty catheters in which the tube 2 has a coaxial geometrical configuration (two tubes of different diameters, of which the smaller is inside the larger) or in which the tube has a single hole, usually in an axial position, defining a single duct both for the guide and pushing wire and for the liquid means which inflates the balloon head 1 ("on the wire" catheters). This is all in accordance with criteria which are widely known in the art and do not therefore need to be described specifically herein.

In practice, some surgical teams have encountered a certain amount of difficulty, with PTA or PTCA catheters of the type described above, in deflating the balloon.

In other words, when the catheter has been inserted and the balloon head 1 has been inflated, a certain amount of difficulty has been encountered in deflating the balloon, either with a view to subsequent re-inflation, for example, when a series of inflation and deflation cycles of the head 1 is to be performed, or - above all - when the catheter is to be removed finally from the patient's body.

Some cases have been reported in which the catheter has actually become stuck, with its head 1 in the inflated condition so that the catheter could not be removed easily from the patient's body.

It has been observed that these deflation problems, which may even involve the catheter becoming stuck, can be attributed to the formation of gas-liquid fragments in the duct 4, that is, in practice, to the break-up of the column of liquid used as a hydraulic means for inflating and deflating the balloon head 1 into a plurality of bodies of liquid separated by gas (air) bubbles. In this respect, it should be noted that, even if the catheter is originally made up with its head 1 completely deflated and virtually evacuated in order subsequently to be filled with the liquid means, it is not generally possible to avoid the presence of a certain amount of air (particularly in the space within in the duct 4 of Figure 2, for example) and this gives rise to the formation of bubbles.

## Objects and summary of the present invention

The object of the present invention is therefore to provide a tube structure which is intrinsically unaffected by problems such as those described above with specific reference to angioplasty catheters.

As already indicated in the introduction to the present description, the solution according to the present invention can, however, be applied in other fields, whenever the formation of gas-liquid fragments can be recognised as a cause of problems which, essentially, make it difficult or impossible to move a liquid which is being transported along a tube.

From this point of view, as will become clear from the description of the theories upon which the invention is based and from the detailed description of a possible embodiment, the aim of the present invention is not completely to prevent the formation of any gas-liquid fragments in the tube, but mainly to limit its extent so that it does not give rise to problems.

The specific characteristics of the invention are described in the following claims.

## Detailed description of the invention

The invention will now be described purely by way of non-limiting example, with reference to the appended drawings, in which:

Figures 1 and 2, which relate to the prior art, have already been described above,

Figure 3, which consists of two parts, indicated a) and b) respectively, shows the physical principle upon which the invention is based,

Figure 4 is a graph showing the relationship between the diameter of a circular tube (on the abscissa) and, on the one hand, the number of gas bubbles which can form inside it as a result of the formation of gas-liquid fragments (curve a) and, on the other hand, the maximum number of bubbles which can be moved along the tube under the effect of a given pressure gradient (curve b),

Figure 5 is a view substantially corresponding to that of Figure 2, showing a tube structure according to the invention,

Figures 6 and 7 show the specific characteristics assumed by the meniscus of a bubble of liquid within a tube having the structure according to Figure 5.

## The theory upon which the invention is based

For a better understanding of the invention, it seems important to provide a brief description of the theories upon which it is based.

In general, a severe obstruction or blockage of the transportation through a tube of a liquid acting as a hydraulic means which, as regards the liquid itself, is manifested by a capillarity effect (for example, in the case of a catheter such as that shown in Figures 1 and 2) could be attributed to at least four different phenomena, that is:

- the collapse of the walls of the tube as a result of the bending or twisting thereof,
- considerable changes in the hydraulic means, for example, in the case of a contrast liquid used as a hydraulic means in a PTA or PTCA catheter, the precipitation/flocculation of the contrast medium caused by high pressure gradients in the discontinuous portions along the catheter,
- the presence of flaps either in the region in which the tube is connected to the balloon head (indicated 1 in Figure 1) or in correspondence with the Y connector (indicated 7, also in Figure 1), or
- the formation of gas-liquid fragments in the tube.

Experiments carried out by the Applicant show that the first three causes actually have no effect, particularly in tubes with diameters of the order of those used in PTA or PTCA catheters (for example, 300 ÷ 500 microns), whereas the formation of gas-liquid fragments in the tube is a determining factor.

Specifically, once gas-liquid fragments have formed, that is, once a series of drops of "bubbles" of liquid separated by "bubbles" of gas has formed within the tube, in order to move the fragmented body of liquid inside the tube, for example, in order to be able to deflate the balloon head 1, it is necessary to apply a very large sudden pressure-change along the tube. In some cases the pressure required becomes so high that, in practice, it gives rise to the blockage of the tube; this arises, for example, in PTA or PTCA catheters when the sudden pressure-change necessary becomes greater than the vacuum which can be applied to its distal end, which is intrinsically limited to 1 atmosphere minus the vapour pressure of the liquid used.

For a closer understanding of the mechanism which regulates the behaviour of gas-liquid fragments in a tube, it should be noted, firstly, that, in such a fragmentary condition, each drop or bubble of liquid is separated from the adjacent gas bubbles by interfaces, that is, by macroscopic discontinuities, where one phase ends and the other begins.

The interface region is subject to continual change and its equilibrium depends upon forces acting at the molecular level. It should be taken into account that, since the bubbles in question are inside a tube, the material defining the walls of the tube (for example, polyethylene) also plays a part in the mechanism, giving rise to a three-phase gas-liquid-solid system.

The conditions affecting molecules in the interface region differ from those affecting molecules in the body of the corresponding medium and this gives rise to a difference in the force acting parallel to the surface. As is well known, this force per unit length is defined as surface tension.

It is also known that, when a drop of liquid is placed on a solid surface, an angle of equilibrium is established between the drop and the surface. This angle is known as the angle of contact and constitutes a measure of the ability of the liquid to wet the surface. When the angle of contact is close to zero, the contact area increases and the liquid spreads out on the solid surface (that is, it wets the solid).

It can be shown experimentally that the phenomenon described above is subject to intrinsic hysteresis. In practice, if small additional volumes of liquid are added to the drop of liquid, the angle of contact alters, displaying hysteresis; it should be noted that a change (either an increase or a decrease) in the amount of liquid in the drop gives rise to a change in the angle of contact before a corresponding sudden change takes place in the contact area.

The phenomenon, commonly defined as capillarity, is also known; this can be displayed by inserting a capillary tube vertically into a liquid and observing that the level reached by the liquid inside the tube differs (upwards or downwards) from the external liquid level by a quantity H which, in the case of a tube with a circular cross-section, can be expressed by the equation:

$$H = \frac{2\sigma.\cos\theta}{\rho\,rg} \qquad (1)$$

where $\sigma$ is the surface tension of the liquid, r is the radius of the tube, $\rho$ is the density of the liquid, $\theta$ is the angle of contact, and g is the acceleration due to gravity, to which the system is subject.

Moreover, the air-liquid interface inside the capillary tube is not flat, but assumes a dished shape of height h; this surface is known as the meniscus. If other factors are left out of consideration, it may be noted that, in the case of a tube with a circular cross-section, the height "h" of the meniscus is inversely proportional to the radius r of the tube (see Figure 3a).

In general, with reference to tubes having non-circular cross-sections, it can be shown that the height of the meniscus is inversely proportional to the distance between the walls of the tube (see Figure 3b, relating to the case of a dihedron D).

If the considerations set out above are applied to gas-liquid fragments in a tube (with capillarity characteristics), it can be seen that, in the absence of pressure applied along the tube, the bubbles or drops of liquid are in equilibrium and their interfaces are inclined at a certain angle of contact to the inner surface of the tube.

If a pressure gradient is applied by a pumping element (for example, a syringe) the hysteresis of the angles of contact will ensure that the drops (that is, their interface surfaces) are first deformed and that the drops of liquid are displaced and the liquid consequently actually transported along the tube only subsequently - when a critical angle of contact has been reached.

In other words, this means that, in order to be able effectively to move fragmented liquid along the tube, it is necessary first of all to apply to each drop of fragmented liquid a sudden change in pressure such as to achieve the critical angle of contact.

In particular (again with reference to a tube of circular cross-section), the force f which acts on the surface of the liquid of a single drop can be expressed in the form:

$$f = p.A = p.\pi.r^2 \qquad (2)$$

where p is the pressure, A is the surface area, and r is the radius of the tube. Because of the hysteresis of the angle of contact, this force must be greater than the longitudinal component of the force f' which is required to displace the gas-liquid-solid interface, and which can be expressed by the equation:

$$f' = 2 \pi r \sigma \{Cos(\theta - \Delta\theta^-) - cos(0 + \Delta\theta^+)\} \qquad (3)$$

where $\sigma$ is the surface tension, $\theta$ is the angle of contact, and $\Delta\theta^+$ and $\Delta\theta^-$ are two different factors which express the hysteresis phenomenon.

If N drops are to be displaced in a tube, it is necessary to apply a sudden pressure-change $\Delta_p$ through the tube, such that

$$\Delta P > N. \Delta p \qquad (4)$$

where $\Delta p$ is the pressure gradient required to move an individual drop.

A development of equation 4 gives the following:

$$\Delta P \geq N. \Delta p = \frac{N2\sigma \{Cos(\theta - \Delta\theta^-) - cos(\theta + \Delta\theta^+)\}}{r} \qquad (5)$$

According to the characteristics of the three-phase (tube/gas/liquid) system, the pressure gradient necessary to displace a fragmented gas-liquid system therefore increases with the number N of bubbles (of liquid or of gas, which are equivalent) and is inversely proportional to the radius of the tube. If the radius is increased, a lower $\Delta$ P is required in order to move a bubble, that is, a larger number of bubbles can be moved by a given $\Delta$ P.

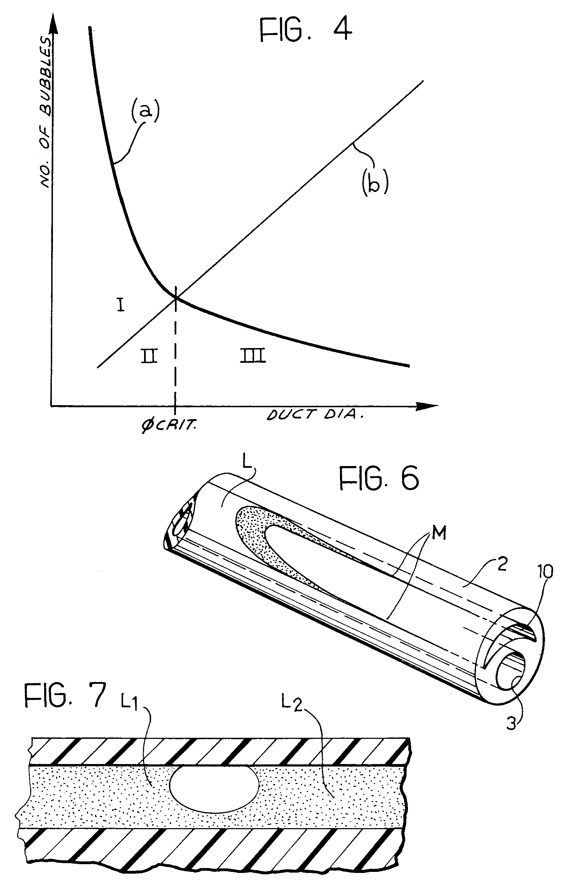

This condition is expressed by the straight line b) of Figure 4, which shows that the number N of bubbles which can be moved by the application of a given pressure gradient to the tube increases linearly with the diameter of the tube.

In practice, in a polyethylene catheter tube of circular cross-section with a diameter of 280 microns (0.012"), theoretical calculations and experimental tests show that, in order to move a drop of a solution of a contrast liquid having characteristics approximately comparable to those of water, a sudden change in pressure of approximately 1/100 - 1/150 of an atmosphere must be applied, which means that up to 100 - 150 bubbles can be moved by a sudden pressure change of 1 atm.

Another factor which it is important to assess is the maximum number of bubbles (of gas or liquid, since the fragmented mixture is simply an alternating sequence of gas and liquid bubbles) which can form within the tube. Clearly, this number is correlated to the height h of the meniscus of each bubble of liquid and consequently to the angle of contact and the geometrical shape of the duct in the tube.

If it is assumed that a circular duct is involved, the meniscus assumes a spherical, dished shape, the height h of which can be expressed by the equation:

$$h = r.\left(\frac{1}{Sin\,\theta} - \frac{Cos\,\theta}{Sin\,\theta}\right) \qquad (6)$$

where r is the radius of the tube and $\theta$ is the angle of contact, in other words, for a given angle of contact,

$$h = a.r \qquad (7)$$

The length L of the smallest bubble which can form inside a circular tube will therefore be 2h.

The maximum number of bubbles which can form inside the tube can thus be expressed as:

$$N = L/2h = L/2.a.r \qquad (8)$$

where L is the overall length of the tube. In practice, the relationship between the number of bubbles and the radius of the tube is hyperbolic, as shown by the curve a) of Figure 4.

With reference to a polyethylene catheter of circular cross-section and 135 cm long, and a contrast fluid having characteristics comparable to those of water, the equations given above give the values listed in the following table:

| Tube diameter (microns) | (inches) | Maximum number of bubbles where $\theta$ = 65 degrees |
|---|---|---|
| 300 | (0.012") | 3530 |
| 380 | (0.015") | 2780 |
| 510 | (0.020") | 2080 |
| 760 | (0.030") | 1390 |
| 1020 | (0.040") | 1040 |

The considerations dealt with above apply specifically to a tube of circular cross-section.

The general result can, however, also be extrapolated to tubes with other cross-sections, in the sense that the number of bubbles which can form in the tube decreases as the height h of the meniscus increases. As has been shown above - see equation (1) in particular - the height of the meniscus is inversely proportional to the distance between facing walls of the tube.

The combined graph a - b of Figure 4 can be interpreted by considering the area below the hyberpola a to be divided theoretically into three regions indicated I, II and III, respectively.

The region I, which is located above the straight line b (which defines the maximum number of bubbles which can be moved inside the tube by a given pressure), defines the conditions in which a blockage of the gas and liquid fragments takes place inside the tube, that is, the region (which corresponds to tube diameters smaller than a critical value $\phi$ crit. defined by the intersection of the hyperbola a and the straight line b) in which the number of bubbles in the tube is greater than the number of bubbles which can be moved along it.

For example, in the case of PTA or PTCA catheters, the selection of a tube diameter smaller than the critical diameter $\phi$ crit. means that a blockage may occur (that is, in practice, it may no longer be possible to deflate the balloon head of the catheter).

The foregoing also applies substantially to the region indicated II in the graph of Figure 4. This region (which is located below the straight line b), corresponds to situations in which the number of bubbles which have formed inside the catheter is very small and, in practice, actually remains below the values defined by the straight line b. Clearly, however, a catheter operating in such conditions is in any case still liable to blockage.

On the other hand, this risk can safely be eliminated if the catheter is formed in a manner such that it falls within the region indicated III, that is, so as to have a tube diameter larger than the critical value $\phi$ crit. Under these conditions, the maximum number of bubbles which can form within the tube is certain always to be smaller than the maximum number of bubbles which can be moved along the tube by a suitable pressure. The region III therefore constitutes a region corresponding to safe operation of the catheter.

The graph of Figure 4 expresses (with reference to a tube of circular cross-section) one of the basic principles of the present invention, that is, the principle according to which - in order to prevent blockages caused by the formation of gas-liquid fragments in a tube - the number of bubbles which can form within the tube must always be smaller than the maximum number of bubbles which can be moved along the tube by a given pressure gradient.

The graph of Figure 4 shows that this result can be achieved in a circular tube if the diameter of the tube is kept larger than a critical minimum diameter $\phi$ crit.

With reference to a possible balloon-head deflation pressure of the order of 1 atmosphere, for polyethylene PTA or PTCA catheters operated by liquids consisting of normal radiographic contrast media, the critical diameter $\phi$ crit. in question is approximately 1.2 mm.

Due to dimensional requirements, in many cases, it may not, however, always be possible to impose this condition on the diameter (and the dimensions in general) of the tube. Neither does it seem to be necessary, however; as has already been seen, the fact that the maximum number of bubbles which can form within the tube is inversely proportional to the diameter of the tube itself results from the fact that the maximum number is actually linked to the height of the menisci of the bubbles by an inverse law; in other words, the maximum number of bubbles which can form within the tube can be reduced by increasing the heights of their menisci.

An indication as to how the required heights of the menisci can be achieved can be derived from an observation of the shape and considerable height h of the meniscus in the case of a dihedron (see Figure 3b), the height h of which is determined by the convergence of its constituent planes near the pointed corner region.

As will become clearer from the following, in the currently-preferred embodiment, the object of the invention is actually to reduce the maximum number of bubbles by using this phenomenon.

Detailed description of an embodiment of the invention

Figure 5 is a cross-section of the tube 2 of a PTA or PTCA catheter having a structure of the type shown in Figure 1; Figure 5 therefore corresponds substantially to the cross-section II-II shown in Figure 2.

In particular, Figure 5 shows how, in a catheter according to the invention, whilst the circular cross-section of the duct 3 for the passage of the guide wire has been kept substantially unaltered, the duct for the passage of the hydraulic fluid (the hole indicated 10) can be modified so as to assume a general crescent shape. The duct 10 thus has a central region 11 in which the distance between the facing walls (which are convex and concave, respectively, with approximately circular, arcuate shapes) is of the order of 0.3 - 0.4 mm, that is, corresponding, in practice, to the diameter of the duct 4 of Figure 2. This distance is then reduced in the end regions 12 so as to form two pointed end regions in which the facing walls gradually converge at an angle $\alpha$ which is smaller than or equal to about 30°, and are finally joined together by a circular end region 13 having a radius of the order, for example, of 0.1 - 0.05 mm. In general, this radius is determined mainly by the technological limitations inherent in the production of the tube 2 (usually by the extrusion of polyethylene). In any case, it can definitely be considered possible to reduce the radius to even smaller values (this is particularly true in the case of applications other than catheters, in which different materials such as, for example, metals, are used).

The overall effect of the crescent shape of the cross-section of the duct 10 is shown schematically in Figure 6 which shows the shape assumed by a quantity of contrast liquid L admitted to a duct having a cross-section of this type.

As can be seen, in the central region of the duct, the liquid L forms a meniscus, the height of which is scarcely perceptible. In the two end regions 12 of the crescent, however, the liquid L forms two menisci M which extend like needles or prongs so that the body of liquid L is generally fork-shaped.

By using a contrast liquid such as Renographin or Iopamiro as the liquid L in a tube with a duct 10 having the dimensional characteristics described above, in a polyethylene catheter tube, the Applicants have consistently been able to measure the formation of menisci M of heights greater than 2 - 2.5 mm.

This drastically reduces the number of bubbles which can form in the tube. For example, in a catheter 135 cm long, this number falls to approximately 270 ÷ 330, compared with an ability to move at least 330 ÷ 500 bubbles along the duct by a sudden pressure change of 1 atmosphere.

The effect of this tube structure as regards the formation of any gas-liquid fragments in the tube is shown in Figure 7. This drawing shows how, although two adjacent bodies of fluid L1, L2 in a tube having the characteristics indicated in Figure 4 are separated in an upper region V, they remain in contact with one another by means of their respective menisci so that, in practice, the continuity of the column of liquid is unbroken. In these conditions, it can be seen that fragmentation of the type described above cannot actually be said to occur; this is because, although the column of liquid incorporates quantities of air, the volumes of which may vary to a certain extent under the effect of a pressure gradient, it is not actually subject to the formation of gas-fluid interfaces which could give rise to hysteresis, or to the blockage of the movement of the liquid along the tube, in general.

The Applicants have also found that the performance of the catheter remains unchanged, without any evidence of blockages, even when it is knotted, for example, with up to three very tight knots in a catheter tube 135 cm long.

Naturally, the principle of the invention remaining the same, the details of construction and forms of embodiment may be varied widely with respect to those described and illustrated, without thereby departing from the scope of the present invention. This applies in particular to the shape of the cross-section of the duct which, instead of the crescent shape described by way of example, may be of another shape, for example it may be drop-shaped or of a lenticular shape, etc., so as to ensure the presence of sharp pointed regions which favour the formation of menisci of considerable extent.

A particular embodiment of the invention may consist of a circular hole in which a wire of smaller diameter is inserted so as to bear against the wall of the hole.

**Claims**

1. A tube (2) for transporting, under the effect of a given pressure gradient applied (6) along a tube, a liquid which may contain gas and may therefore give rise to the formation of gas-fluid fragments constituted by a plurality of bubbles of liquid separated by gas bubbles in the tube, characterised in that it has a cross-section (10) such that the number of bubbles which can form in the tube (2) is lower than the maximum number of bubbles which can be moved along the tube (2) by the pressure gradient.

2. A tube according to Claim 1, characterised in that it has a circular cross-section, the diameter of which is always larger than a minimum critical diameter ($\phi$ crit.).

3. A tube according to Claim 1, characterised in that it includes a main portion (11) and at least one edge portion (12) in which the walls defining the tube converge so as to cause the liquid to form a meniscus M of a height substantially greater than that of the meniscus formed by the liquid in the main portion (11).

4. A tube according to Claim 3, characterised in that the cross-section of the tube has at least one pointed edge portion (12) defining a given angle ($\alpha$) which is preferably less than or equal to about 30°.

5. A tube according to Claim 3 or Claim 4, characterised in that the edge portion has a radius of curvature of the order of 0.15 mm or less.

6. A tube according to any one of Claims 3 to 5, characterised in that it has a generally crescent-shaped cross-section (11, 12).

7. A tube according to Claim 6, characterised in that the crescent-shaped tube has a main portion (11) about 0.3 ÷ 0.4 mm wide and two pointed edge portions (12) with radii of curvature of the order of 0.05 ÷ 0.1 mm.

8. The use of a tube according to any one of the preceding claims as a catheter tube (2), particularly an angioplasty catheter tube.

FIG. 1  *PRIOR ART*

FIG. 2  *PRIOR ART*

FIG. 5

FIG. 3

**a**

**b**

FIG. 4

FIG. 6

FIG. 7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 850 969 (JACKSON) <br> * column 3, line 9 - line 17; figures 9,10 * | 1-8 | A61M25/10 |
| X | US-A-4 755 176 (PATEL) <br> * column 2, line 16 - line 23; figures * | 1-8 | |
| X | US-A-3 499 435 (ROCKWELL ET AL) <br> * figures * | 1-8 | |

TECHNICAL FIELDS
SEARCHED (Int. Cl.5)

A61M

The present search report has been drawn up for all claims

| Place of search | Date of completion of the search | Examiner |
|---|---|---|
| THE HAGUE | 21 OCTOBER 1992 | CLARKSON P. |

EPO FORM 1503 03.82 (P0401)